# EUROPEAN PATENT SPECIFICATION

(11) **EP 3 768 699 B1**
(45) Date of publication and mention of the grant of the patent: **12.11.2025**
(21) Application number: 19722960.2
(22) Date of filing: 22.03.2019
(51) Int. Cl.: C07K 14/54, C12N 5/0783, A61K 40/11, A61K 40/22, A61K 40/41

(54) **GENETICALLY REPROGRAMMED TREGS EXPRESSING MEMBRANE-BOUND IL-10**
GENETISCH NEU PROGRAMMIERTE TREGS, DIE MEMBRANGEBUNDENES IL-10 EXPRIMIEREN
TREGS GÉNÉTIQUEMENT REPROGRAMMÉS EXPRIMANT IL-10 LIÉ À UNE MEMBRANE

(30) Priority: 23.03.2018 US 201862647084 P
(43) Date of publication of application: 27.01.2021
(73) Proprietor: Gavish-Galilee Bio Applications Ltd., 1101602 Kiryat Shmona (IL)
(72) Inventor: GROSS, Gideon, 1292200 Moshav Almagor (IL); WEINSTEIN-MAROM, Hadas, 1223500 Kibbutz Dafna (IL); KRONER, Amit, 1080300 Kibbutz Nir David (IL)
(74) Representative: Boult Wade Tennant LLP
(86) International application number: PCT/IL2019/050324
(87) International publication number: WO 2019/180724

(56) References cited:
- WO-A1-2012/069627
- WO-A2-2008/095141
- GRAZIA LOCAFARO ET AL: "IL-10-Engineered Human CD4 + Tr1 Cells Eliminate Myeloid Leukemia in an HLA Class I-Dependent Mechanism", MOLECULAR THERAPY, vol. 25, no. 10, 1 October 2017 (2017-10-01), GB, pages 2254 - 2269, XP055592839, ISSN: 1525-0016, DOI: 10.1016/j.ymthe.2017.06.029
- GRAZIA ANDOLFI ET AL: "Enforced IL-10 Expression Confers Type 1 Regulatory T Cell (Tr1) Phenotype and Function to Human CD4+ T Cells", MOLECULAR THERAPY, vol. 20, no. 9, 1 September 2012 (2012-09-01), GB, pages 1778 - 1790, XP055592834, ISSN: 1525-0016, DOI: 10.1038/mt.2012.71
- JETHWA HANNAH ET AL: "Use of gene-modified regulatory T-cells to control autoimmune and alloimmune pathology: Is now the right time?", CLINICAL IMMUNOLOGY, ACADEMIC PRESS, US, vol. 150, no. 1, 16 November 2013 (2013-11-16), pages 51 - 63, XP028671363, ISSN: 1521-6616, DOI: 10.1016/J.CLIM.2013.11.004
- NICOLA GAGLIANI ET AL: "Coexpression of CD49b and LAG-3 identifies human and mouse T regulatory type 1 cells", NATURE MEDICINE, vol. 19, no. 6, 28 April 2013 (2013-04-28), pages 739 - 746, XP055182035, ISSN: 1078-8956, DOI: 10.1038/nm.3179
- MILLS KINGSTON H G ET AL: "Antigen-specific regulatory T cells--their induction and role in infection.", SEMINARS IN IMMUNOLOGY APR 2004, vol. 16, no. 2, April 2004 (2004-04-01), pages 107 - 117, XP002791704, ISSN: 1044-5323
- SABAT R ET AL: "Biology of interleukin-10", CYTOKINE AND GROWTH FACTOR REVIEWS, ELSEVIER LTD, GB, vol. 21, no. 5, 1 October 2010 (2010-10-01), pages 331 - 344, XP027543794, ISSN: 1359-6101, [retrieved on 20101001]
- FUJIO KEISHI ET AL: "The Family of IL-10-Secreting CD4(+) T Cells", ADVANCES IN IMMUNO, ACADEMIC PRESS INC., NEW YORK, NY, US, 1 January 2010 (2010-01-01), pages 99 - 130, XP009192122, ISSN: 0065-2776

## Description

### FIELD OF THE INVENTION

The present invention relates in general to genetically reprogrammed regulatory T cells expressing membrane-bound IL10 and to said genetically reprogrammed regulatory T cells expressing membrane-bound IL10 for use in treating diseases manifested in excessive activity of the immune system.

### BACKGROUND OF THE INVENTION

Harnessing CD4 regulatory T cells (Tregs) for suppressing local inflammation and restoring immunological balance holds great promise in the treatment of pathologies as diverse as autoimmune diseases, inflammatory bowel diseases, allergies, atherosclerosis, transplant rejection, graft-versus-host disease and more. However, Tregs, either natural (nTregs) or induced (iTregs) form only a minor fraction in the entire human CD4 T cell population. Consequently, there is an urgent need for the development of Treg-based therapies for recruiting, inducing, or engineering autologous or allogeneic Tregs at adequate numbers and stable phenotype which are critical for clinical efficacy and safety of treatment.

### SUMMARY OF INVENTION

The invention is set out in the appended claims.

In one aspect, the present invention provides an isolated nucleic acid molecule comprising a nucleotide sequence encoding a homodimeric IL-10 linked to a transmembrane-intracellular stretch, optionally through a flexible hinge, referred to herein as mem-IL10.

In a different aspect, the present invention provides a composition comprising the nucleic acid molecule comprising a nucleotide sequence encoding a homodimeric IL-10 linked to a transmembrane-intracellular stretch as defined herein.

In a further aspect, the present invention provides a viral vector comprising any one of the nucleic acid molecules comprising a nucleotide sequence encoding a homodimeric IL-10 linked to a transmembrane-intracellular stretch as defined above.

In another aspect, the present invention provides a composition comprising the viral vector as defined above.

In still another aspect, the present invention provides a mammalian regulatory T cell (Treg) comprising any one of the nucleic acid molecules as defined above, or the viral vector as defined above.

In yet an additional aspect, the present invention provides an ex vivo method of preparing allogeneic or autologous Tregs with a stable Tr1 phenotype, the method comprising contacting CD4 T cells with the nucleic acid molecule comprising a nucleotide sequence encoding a homodimeric IL-10 as defined above, or a viral vector comprising it, thereby endowing said CD4 T cells with a stable Tr1 phenotype, and thus preparing Tregs with a stable Tr1.

In certain embodiments, the present invention provides a mammalian Treg expressing on its surface a homodimeric IL-10 as defined above, for use in treating or preventing a disease, disorder or condition manifested in excessive or otherwise unwanted activity of the immune system, such as an autoimmune disease, allergy, asthma, and organ and bone marrow transplantation.

### BRIEF DESCRIPTION OF DRAWINGS

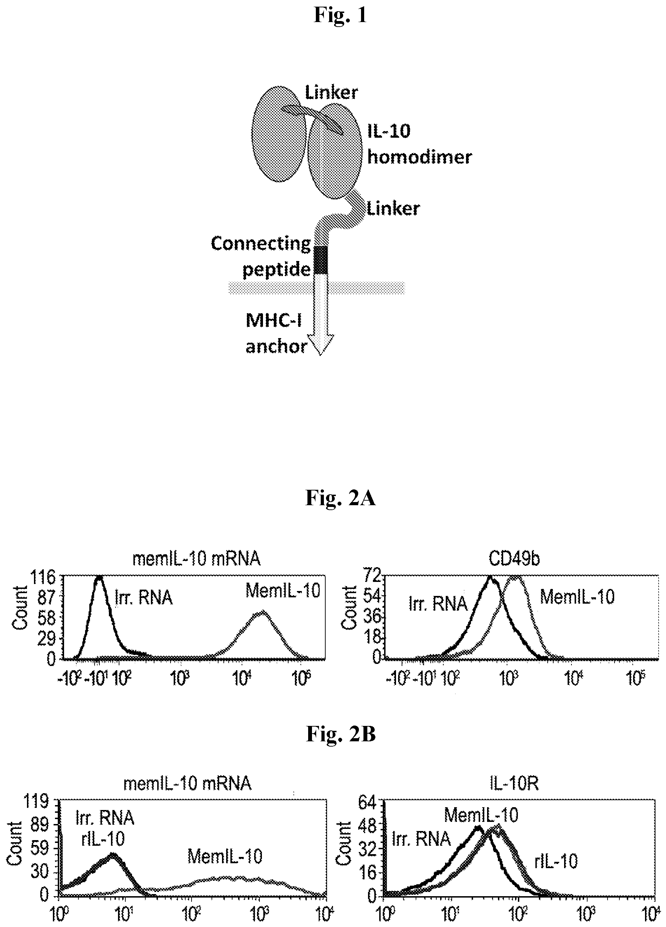

**Fig. 1** depicts a schematic presentation of membrane-anchored homodimeric IL-10.
**Figs. 2A-2D** show analysis of memIL-10 expression in T cells and its effect on IL-10 receptor (IL-10R) and CD49b. Human Jurkat or primary, peripheral blood lymphocyte-derived CD4 T cells **(A, B)** and mouse B3Z or NOD splenic CD4 T cells **(C, D)** were electroporated with 10 µg of *in-vitro* transcribed mRNA encoding human or mouse memIL-10, respectively. Cells were analyzed by flow cytometry 24 hours **(A-C)** or 48 hours **(D,** left and right) post-transfection. Human or mouse memIL-10 and IL-10R and human CD49b were analyzed by monoclonal antibodies specific to the respective human or mouse proteins, respectively.

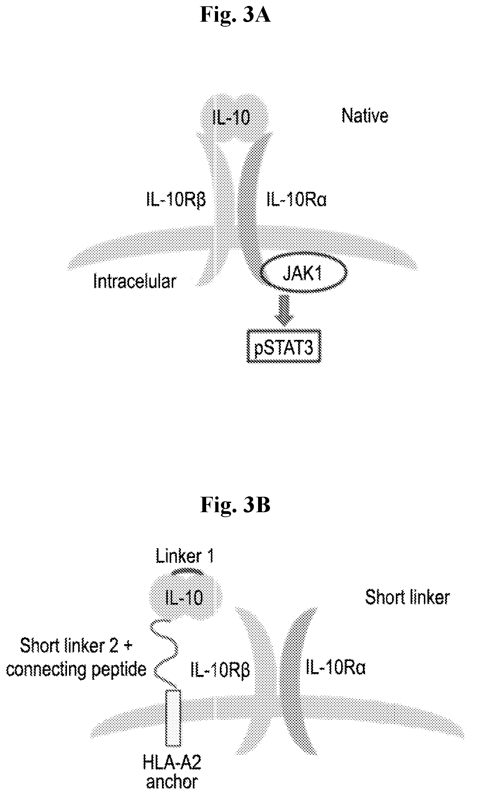

**Figs. 3A-D** depict schematic presentations of native IL-10 homodimer bound to its cell surface receptor **(A)** and of the three membrane-anchored derivatives of IL-10 (mem-IL10): **(B)** mem-IL10 with short linker; **(C)** mem-IL10 with long linker; and **(D)** mem-IL10 linked to IL-10Rβ (IL-10Rβ fusion).

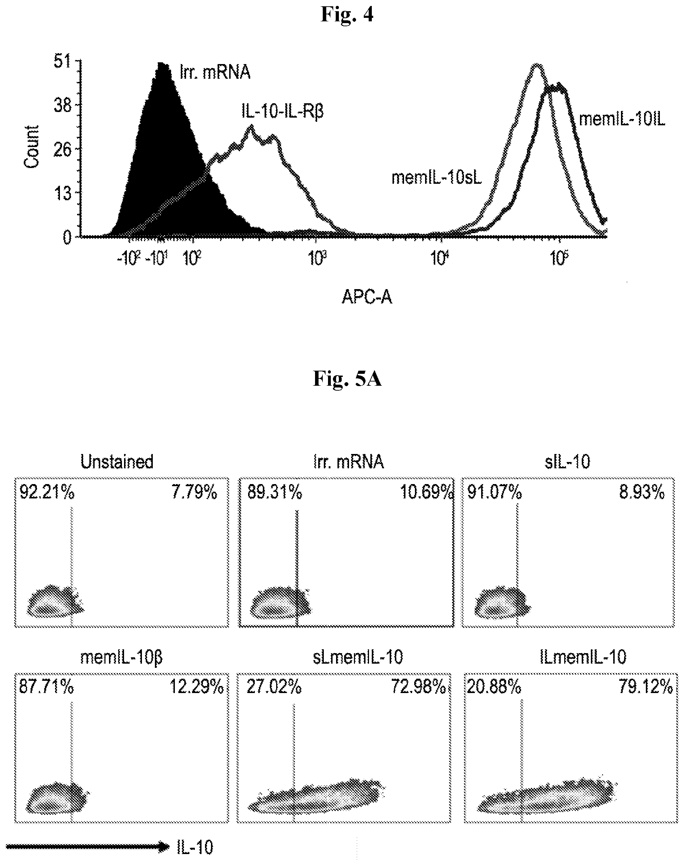

**Fig. 4** shows cell surface expression of the three memIL-10 derivatives in Jurkat cells 24 hours post-mRNA electroporation. Human Jurkat CD4 T cells were electroporated with 10 µg of each of the indicated mRNAs (sL and lL stand for short and long linker, respectively). Twenty four hours cells were analyzed by flow cytometry for surface expression of IL-10.
**Figs. 5A-C** show that memII,-10 expression in CD4 T cells induces spontaneous phosphorylation of STAT3. Mouse CD4 T cells were either electroporated with irrelevant mRNA (Irr. mRNA), mRNA encoding short linker memIL-10 (sLmemIL-10), long linker memIL-10 (lLmemIL-10) or IL-10 linked to the IL-10Rβ chain (memIL-10Rβ) or treated with soluble recombinant IL-10 (sIL-10) at 20 ng/ml. Twenty four hours later cells were subjected to flow cytometry analysis for surface IL-10 **(A),** surface IL-10Rα chain **(B)** or intracellularly for phosphorylated STAT3 (pSTAT3) **(C).**

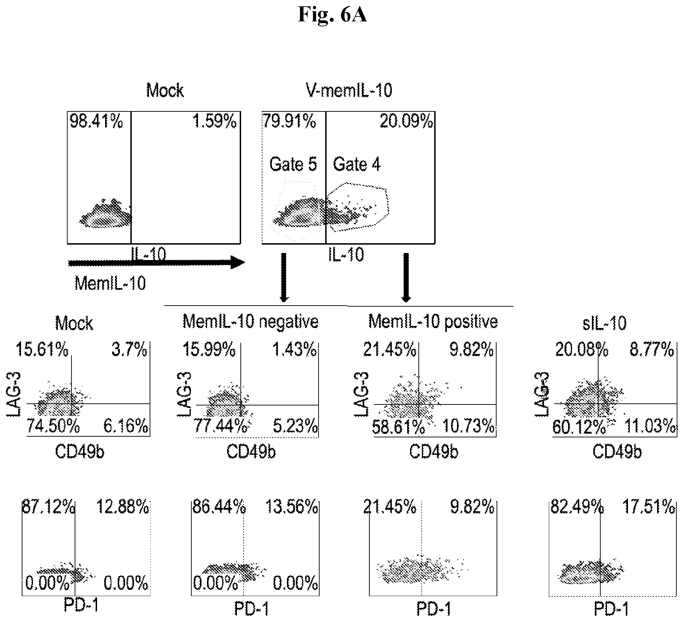

**Figs. 6A-B** show analysis of retrovirally transduced mouse CD4 T cells expressing memIL-10. Phenotypic analysis of short-linker memIL-10-ransduced mouse CD4 T cells (v-memIL-10), 48 hours **(A)** and 6 days **(B)** post-transduction. Analysis was performed in parallel on memIL-10(+) and memIL-10(-) cells growing in the same cell culture, staining for LAG-3, CD49b and PD-1. As a positive control non-transduced cells were treated with soluble IL-10 (sIL-10). Mock, cells treated with identical protocol as retrovirally transduced cells but without exposure to viral particles.

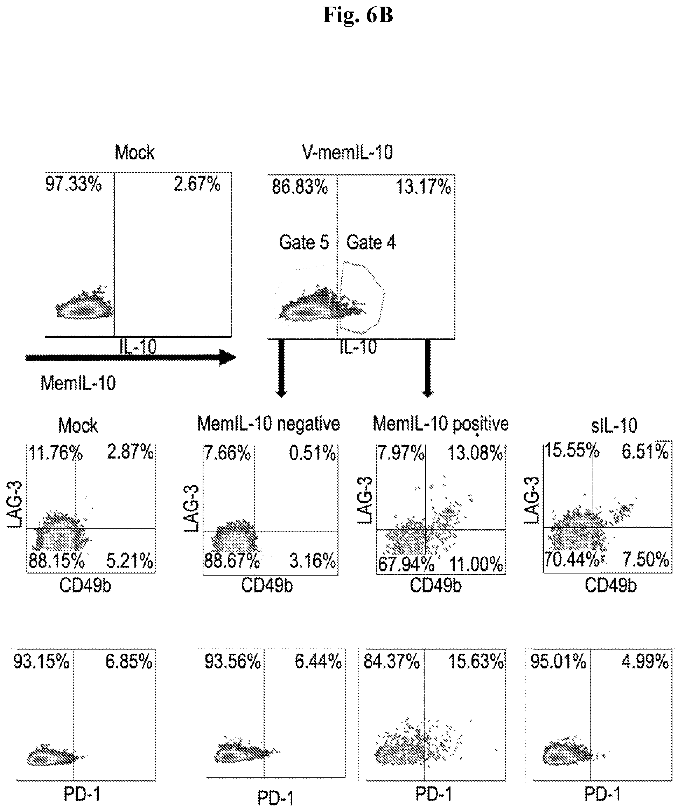

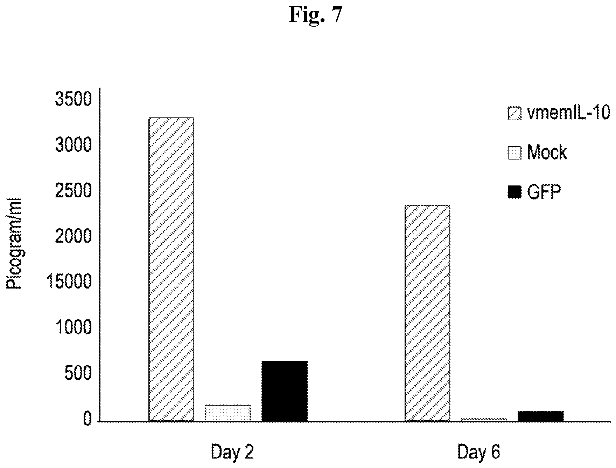

**Fig. 7** shows secretion of IL-10 by activated, memIL-10 transduced mouse CD4 T cells. Cells from the same experiment as in **Fig. 6** were stimulated by an anti-TCR-CD3 mAb (2C11) and their growth medium was subjected to an IL-10 ELISA. Mock- and GFP-transduced T cells serves as negative controls.

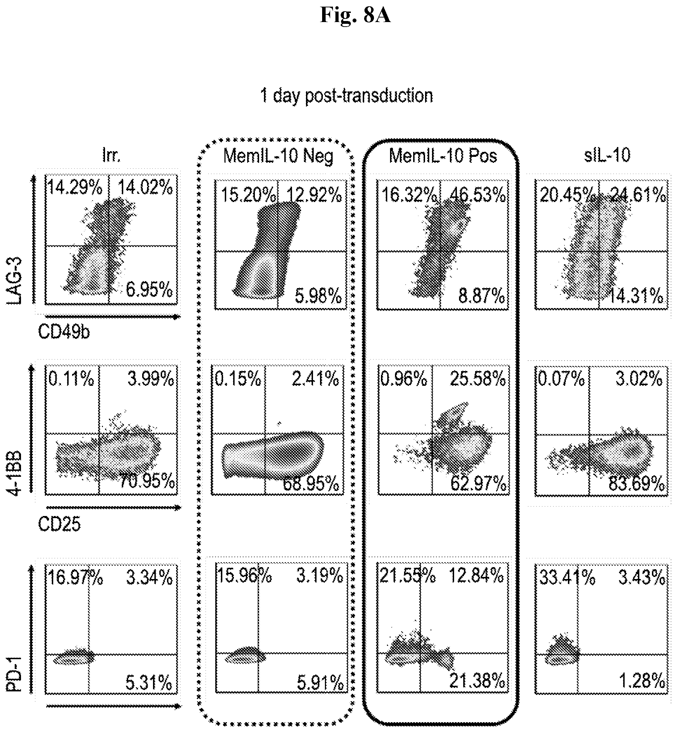

**Figs. 8A-C** show phenotypic characterization of memIL-10 transduced human CD4 T cells. CD4 T cells were isolated by magnetic beads from peripheral blood mononuclear cells prepared from a blood sample of a healthy donor. Cells were grown in the presence of the anti-CD3 and anti-CD28 antibodies and IL-2 to the desired number and transduced with recombinant retrovirus encoding memIL-10 or an irrelevant gene (Irr.), or treated with soluble IL-10 (sIL-10). Cells were grown in the presence of IL-2 and samples were taken for flow cytometry analysis for the indicated cell surface markers at day 1 **(A),** day 5 **(B)** and day 18 **(C).** At day 18 non-transduced Tregs were added to the analysis for comparison of cell surface markers. At each time point cells expressing memIL-10 (Pos, solid frame)) were analyzed side by side with cells from the same culture which do not express IL-10 (Neg, dotted frame).

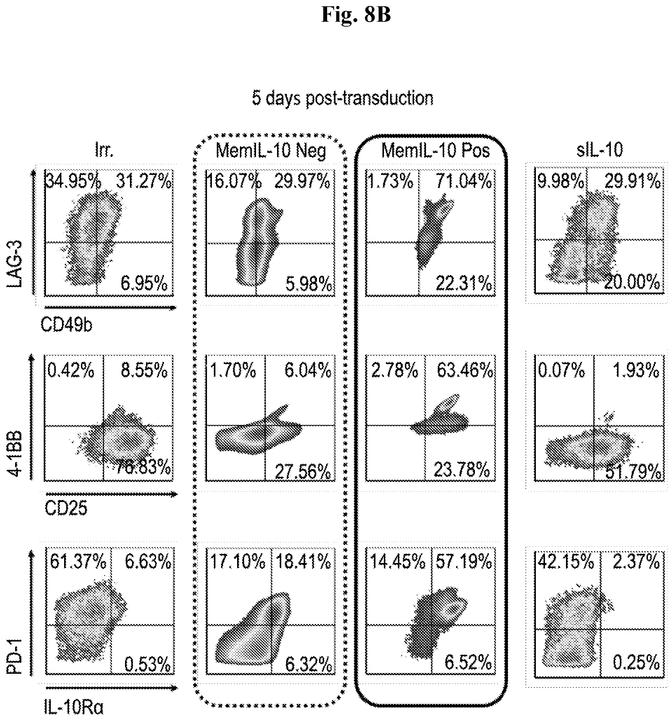

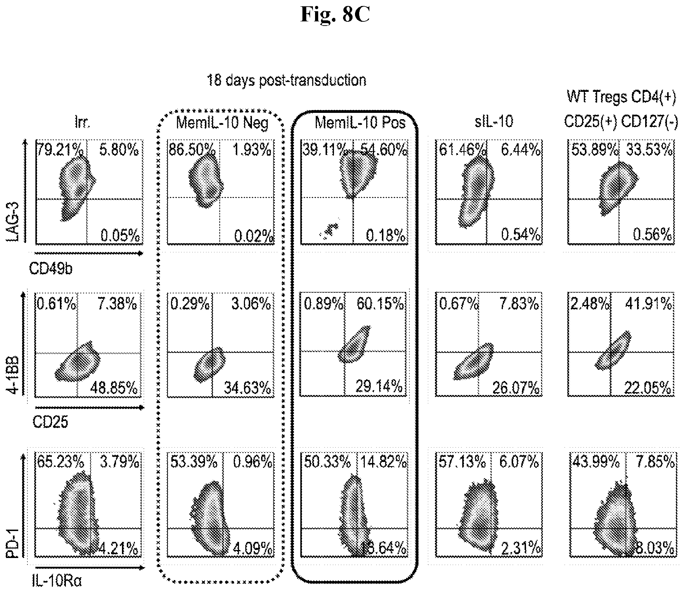

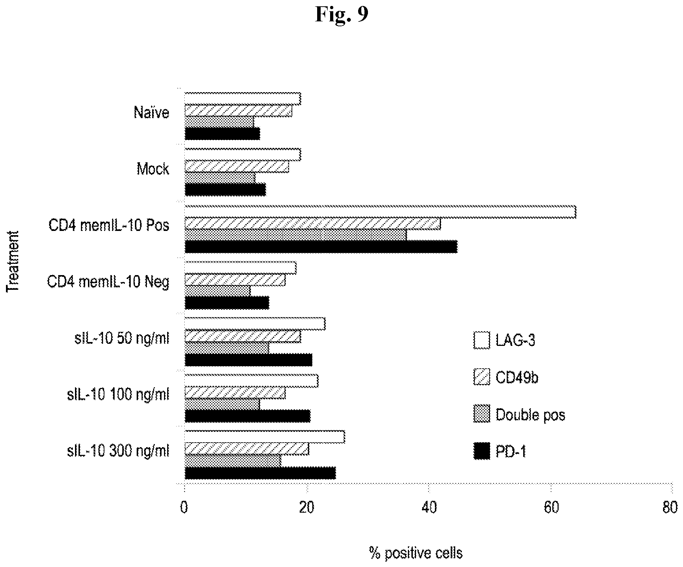

**Fig. 9** shows a second experiment phenotyping memIL-10-transduced human CD4 T cells. Cells were prepared and transduced with memIL-10 and analyzed 4 days later for the indicated markers as described in the legend to **Fig. 8****.** Non-transduced (Naive) and mock-transduced (Mock) CD4 cells served as negative controls. MemIL-10 positive cells were compared to memII,-10 negative cells from the same culture as well as to naive CD4 T cells grown in the presence of 50, 100 or 300 ng/ml sIL-10. Shown are % of positively stained cell in each sample. Double pos, % of cells stained positive for LAG-3 and CD49b.

### DETAILED DESCRIPTION OF THE INVENTION

It has been found in accordance with the present invention that genetically reprogramming T cells to constitutively express membrane-bound IL-10 confers a stable Tr1 phenotype to the T cells.

The type of Treg cell selected is of critical importance for successful clinical implementation. Tr1 cells are a subset of CD4(+) FoxP3(+/-) Tregs which are induced in the periphery in a TCR- and antigen-specific manner upon chronic exposure to antigen on dendritic cells in the presence of IL-10 (1, 2). These cells are characterized by a non-proliferative (anergic) state, high production of IL-10 and TGF-β but only minimally of IL-2 and none of IL-4 or IL-17 and the ability to suppress effector T cells (Teffs) in a cell-to-cell contact-independent manner. Andolfi et al. demonstrated that the enforced expression of IL-10 in human CD4 T cells, accomplished by lentiviral transduction, was sufficient for endowing these cells with a stable Tr1 phenotype in an autocrine fashion (3). This study also showed that exposure of these cells to IL-2 could temporarily reverse the anergic state of these IL-10-induced Tr1 cells. Importantly, two cell surface markers, CD49b and LAG-3, have been identified, which are stably and selectively co-expressed on human (and mouse) Tr1 cells and allow their isolation and flow cytometry analysis for purity of the cell population (4).

The present invention provides a gene encoding a membrane-anchored derivative of IL-10 (mem-IL10). Native IL-10 is a homodimer (5, 6) and it was found herein that imparting a functional homodimeric configuration on its membrane-anchored form provides an IL-10-driven safe lock guaranteeing permanent preservation of the Tr1 phenotype, while avoiding IL-10 secretion in the absence of antigenic stimulation. Safety wise, as IL-10 does not signal T cell proliferation, the autonomous activation of the IL-10 signaling pathway is not associated with risk of uncontrolled cell growth.

In this invention we achieve an anti-inflammatory effect for imposing immune suppression, for the first time, by modifying Tregs to express membrane IL-10. Furthermore, since IL-10 does not induce T cell proliferation it can be expressed constitutively through stable viral transduction with no risk of inducing autonomous cell proliferation and cellular transformation.

In one aspect, the present invention provides an isolated nucleic acid molecule comprising a nucleotide sequence encoding a homodimeric IL-10 linked to a transmembrane-intracellular stretch, optionally through a flexible hinge, referred to herein as mem-IL10.

In certain embodiments, the isolated nucleic acid molecule does not comprise a nucleotide sequence encoding for additional different proteins except for mem-IL-10, but may comprise additional control elements such as promoters and terminators.

In certain embodiments, the homodimeric IL-10 comprises a first and a second IL-10 monomer connected in a single-chain configuration such that the C-terminus of the first IL-10 monomer is linked to the N-terminus of the second IL-10 monomer via a first flexible linker.

Flexible peptide linkers are well-known in the art. Empirical linkers designed by researchers are generally classified into three categories according to their structures: flexible linkers, rigid linkers, and *in vivo* cleavable linkers as defined e.g. in (7-9).

As stated above, the first linker is a flexible linker and its structure is selected from any one of the linkers disclosed in (7-9). In principle, to provide flexibility, the linkers are generally composed of small, non-polar (e.g. Gly) or polar (e.g. Ser or Thr) amino acids, such an underlying sequence of alternating Gly and Ser residues. Solubility of the linker and associated homodimeric IL-10 may be enhanced by including charged residues; e.g. two positively charged residues (Lys) and one negatively charged residue (Glu). The linker may vary from 2 to 31 amino acids, optimized for each condition so that the linker does not impose any constraints on the conformation or interactions of the linked partners in lengths, such as between 12 and 18 residues.

In certain embodiments, the first flexible linker has the amino acid sequence GSTSGSGKPGSGEGSTKG (SEQ ID NO: 1). In certain embodiments, the first flexible linker is encoded by a nucleotide sequence e.g. as set forth in SEQ ID NO: 2.

In certain embodiments, the flexible hinge comprises a polypeptide selected from the following polypeptides or variants thereof:
- The hinge region of CD8α, (for example as set forth in SEQ ID NO: 3; e.g. encoded by a nucleotide sequence as set forth in SEQ ID NO: 4)
- The hinge region of the heavy chain of IgG (for example as set forth in SEQ ID NO: 5; e.g. encoded by a nucleotide sequence as set forth in SEQ ID NO: 6)
- The hinge region of the heavy chain of IgD (for example as set forth in SEQ ID NO: 7; e.g. encoded by a nucleotide sequence as set forth in SEQ ID NO: 8).
- The extracellular stretch of the IL-10R β chain (as set forth in SEQ ID NO: 9; e.g. encoded by a nucleotide sequence as set forth in SEQ ID NO: 10); and
- A second flexible linker comprising an amino acid sequence of up to 28 amino acids comprising at least one Gly4Ser(Gly3Ser)₂ sequence, e.g. comprising one Gly₄Ser(Gly₃Ser) sequence (SEQ ID NO: 11; for example encoded by a nucleotide sequence as set forth in SEQ ID NO: 12), or two Gly₄Ser(Gly₃Ser) sequences with one or two Ser residues inserted between them.

In certain embodiments, the second flexible linker comprises a 21 amino acid sequence comprising the amino acid sequence Gly₄Ser(Gly₃Ser)₂ (referred to herein as "short linker"; SEQ ID NO: 13; for example encoded by a nucleotide sequence as set forth in SEQ ID NO: 14).

In certain embodiments, the second flexible linker consists of a 28 amino acid spacer comprising the amino acid sequence Gly₄Ser(Gly₃Ser)₂Ser₂(Gly₃Ser)₃ (referred to herein as "long linker"; SEQ ID NO:15; for example encoded by a nucleotide sequence as set forth in SEQ ID NO: 22) and the connecting peptide of SEQ ID NO: 16.

In certain embodiments, the second flexible linker of any one of the above embodiments further comprises an 8 amino acid bridge of the sequence SSQPTIPI (referred to herein as "connecting peptide"; SEQ ID NO: 17; for example encoded by a nucleotide sequence as set forth in SEQ ID NO: 18) derived from the membrane-proximal part of the connecting peptide of HLA-A2.

In certain embodiments, the transmembrane-intracellular stretch of the mem-IL10 is derived from the heavy chain of a human MHC class I molecule selected from an HLA-A, HLA-B or HLA-C molecule, preferably HLA-A2 (as set forth in SEQ ID NO: 19; e.g. encoded by a nucleotide sequence as set forth in SEQ ID NO: 20); human CD28 (as set forth in SEQ ID NO: 21; e.g. encoded by a nucleotide sequence as set forth in SEQ ID NO: 22); or human IL-10R β chain (as set forth in SEQ ID NO: 23; e.g. encoded by a nucleotide sequence as set forth in SEQ ID NO: 24).

In certain embodiments, the amino acid sequence of the complete mem-IL10 comprises or essentially consists of the homodimeric IL-10 linked via the short second flexible linker and the connecting peptide to the transmembrane-intracellular stretch of HLA-A2 as set forth in SEQ ID NO: 25; e.g. encoded by a nucleotide sequence as set forth in SEQ ID NO: 26.

In certain embodiments, the amino acid sequence of the complete mem-IL10 comprises or essentially consists of the homodimeric IL-10 linked via the long second flexible linker and the connecting peptide to the transmembrane-intracellular stretch of HLA-A2 as set forth in SEQ ID NO: 27; e.g. encoded by a nucleotide sequence as set forth in SEQ ID NO: 28).

In certain embodiments, the mem-IL-10 is fused to the IL-10Rβ extracellular domain (for example as set forth in SEQ ID NO: 9) via a second flexible linker, and optionally further to the IL-10Rβ transmembrane & cytosolic domains (for example as set forth in SEQ ID NO: 23).

In certain embodiments, the mem-IL-10 is fused to the N-terminus of an essentially complete IL-10R β chain via the short linker (as set forth in SEQ ID NO: 29; e.g. encoded by a nucleotide sequence as set forth in SEQ ID NO: 23).

The polypeptides making up the mem-IL10 of the present invention that are encoded by the nucleic acid molecules of the invention are not limited to those defined herein by specific amino acid sequences but may also be variants of these oligopeptides or have amino acid sequences that are substantially identical to those disclosed above. A "substantially identical" amino acid sequence as used herein refers to a sequence that differs from a reference sequence by one or more conservative or non-conservative amino acid substitutions, deletions, or insertions, particularly when such a substitution occurs at a site that is not the active site of the molecule, and provided that the polypeptide essentially retains its functional properties. A conservative amino acid substitution, for example, substitutes one amino acid with another of the same class, e.g., substitution of one hydrophobic amino acid with another hydrophobic amino acid, a polar amino acid with another polar amino acid, a basic amino acid with another basic amino acid and an acidic amino acid with another acidic amino acid. One or more amino acids can be deleted from the peptide, thus obtaining a fragment thereof without significantly altering its biological activity.

In certain embodiments, the amino acid sequence of the complete membrane-bound IL-10 or each one of the various sub-regions of the membrane-bound IL-10 as disclosed above i.e. the homodimeric IL-10 in which the first and second IL-10 monomers are connected in a single-chain configuration via a first flexible linker; the first flexible linker *per se,* the flexible hinge; and the transmembrane-intracellular stretch, is at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, or at least 98% identical to SEQ ID NO: 1, 3, 5, 7, 9, 11, 13, 15, 17, 19, 21, 23, 25, 27, or 29.

In certain embodiments, the amino acid sequence of the complete membrane-bound IL-10 or each one of the various sub-regions of the membrane-bound IL-10 as disclosed above i.e. the homodimeric IL-10 in which the first and second IL-10 monomers are connected in a single-chain configuration via a first flexible linker; the first flexible linker *per se,* the flexible hinge; and the transmembrane-intracellular stretch, as well as the whole construct, is 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98, or 99% identical to SEQ ID NO: 1, 3, 5, 7, 9, 11, 13, 15, 17, 19, 21, 23, 25, 27, or 29.

In certain embodiments, the isolated nucleic acid molecule comprises a polynucleotide sequence encoding the complete membrane-bound IL-10 or each one of the various sub-regions of the membrane-bound IL-10 as disclosed above i.e. the homodimeric IL-10 in which the first and second IL-10 monomers are connected in a single-chain configuration via a first flexible linker; the first flexible linker *per se,* the flexible hinge; and the transmembrane-intracellular stretch, as well as the whole construct, that is at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, or at least 98% identical to one of SEQ ID NOs: 2, 4, 6, 8, 10, 12, 14, 16, 18, 20, 22, 24, 26, 28 or 30.

In certain embodiments, the isolated nucleic acid molecule comprises a polynucleotide sequence encoding the complete membrane-bound IL-10 or each one of the various sub-regions of the membrane-bound IL-10 as disclosed above i.e. the homodimeric IL-10 in which the first and second IL-10 monomers are connected in a single-chain configuration via a first flexible linker; the first flexible linker *per se,* the flexible hinge; and the transmembrane-intracellular stretch, as well as the whole construct is 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98, or 99% identical to one of SEQ ID NOs: 2, 4, 6, 8, 10, 12, 14, 16, 18, 20, 22, 24, 26, 28 or 30.

In certain embodiments, the isolated nucleic acid molecule comprises a polynucleotide sequence encoding the complete membrane-bound IL-10 or each one of the various sub-regions of the membrane-bound IL-10 as disclosed above i.e. the homodimeric IL-10 in which the first and second IL-10 monomers are connected in a single-chain configuration via a first flexible linker; the flexible linker *per se,* the flexible hinge; and the transmembrane-intracellular stretch, as well as the whole construct as set forth in one of SEQ ID NOs: 2, 4, 6, 8, 10, 12, 14, 16, 18, 20, 22, 24, 26, 28 or 30.

In a different aspect, the present invention provides a composition comprising the nucleic acid molecule comprising a nucleotide sequence encoding a homodimeric IL-10 linked to a transmembrane-intracellular stretch as defined in any of the above embodiments.

In certain embodiments the nucleic acid molecule is the sole nucleic acid molecule in the composition, i.e. the composition does not comprise additional nucleic acid molecules comprising nucleotide sequences encoding for additional different proteins.

The nucleic acid molecules of the present invention are delivered into T cells for the purpose of enforcing a stable Tr1 phenotype using any well-known method in the field: For example, Matuskova and Durinikova (10) teach that there are two systems for the delivery of transgenes into a cell - viral and non-viral. The non-viral approaches are represented by polymer nanoparticles, lipids, calcium phosphate, electroporation/nucleofection or biolistic delivery of DNA-coated microparticles.

There are two main types of vectors that can be used in accordance with the present invention depending on whether the DNA is integrated into chromatin of the host cell or not. Retroviral vectors such as those derived from gammaretroviruses or lentiviruses persist in the nucleus as integrated provirus and reproduce with cell division. Other types of vectors (e.g. those derived from herpesviruses or adenoviruses) remain in the cell in the episomal form.

Thus, in a further aspect, the present invention provides a viral vector comprising anyone of the nucleic acid molecules comprising a nucleotide sequence encoding a homodimeric IL-10 linked to a transmembrane-intracellular stretch as defined above.

In certain embodiments, the viral vector is selected from a modified virus derived from a virus selected from the group consisting of a retrovirus, lentivirus, gammavirus, adenovirus, adeno-associated virus, pox virus, alphavirus, and herpes virus.

In particular embodiments, the vector is a retrovirus, such as a modified gammavirus, lentivirus, murine stem cell virus, moloney murine leukemia virus, bovine leukaemia virus, Rous sarcoma virus, and spumavirus. In fact, of the 52 clinical trials evaluating CAR-T cell in solid tumors which are listed in (11), 24 use retroviral vectors and 9 use lentiviral vectors. It is also noted that the two FDA-approved CAR products for the treatment of B cell malignancies are Kymriah^{™} (lentiviral vector) and Yescarta^{™} (gamma-retroviral vector). Thus, good candidates for the viral vector of the present invention may be retroviral vectors, lentiviral vectors and gamma-retroviral vectors. For example, the retrovirus may be derived from moloney murine leukemia virus or murine stem cell virus sequences (gamma-retroviral vectors).

In certain embodiments, the nucleic acid molecule is the sole polypeptide encoded by the nucleotide sequence, i.e. the nucleic acid molecule of the viral vector does not encode for additional different proteins, but may comprise additional control elements such as promoters and terminators.

In another aspect, the present invention provides a composition comprising the viral vector as defined above.

In still another aspect, the present invention provides a mammalian regulatory T cell (Treg) comprising any one of the nucleic acid molecules as defined above, or the viral vector as defined above.

In certain embodiments, the mammalian Treg expresses on its surface a homodimeric IL-10 that is linked to a transmembrane-intracellular stretch, optionally through a flexible hinge.

In a certain embodiment, the mammalian Treg is a human Treg.

In certain embodiments, the mammalian Treg has a stable Tr1 phenotype (that is, not losing their regulatory activity (12) exhibiting the cell-surface markers CD49b and LAG-3.

In yet an additional aspect, the present invention provides an ex vivo method of preparing allogeneic or autologous Tregs with a stable Tr1 phenotype, the method comprising contacting CD4 T cells with the nucleic acid molecule comprising a nucleotide sequence encoding a homodimeric IL-10 as defined above, or a viral vector comprising it, thereby endowing said CD4 T cells with a stable Tr1 phenotype, and thus preparing Tregs with a stable Tr1.

Methods for preparing CD4 T cells are well known in the art and may be performed e.g. by the method disclosed below in the Examples section.

Methods for creating recombinant retroviral and lentiviral vectors and using them for transducing T cells are also well-known in the art and are usually performed using commercial kits including packaging cells, plasmids and transfection reagents, which are offered by many companies, including Invitrogen^{®}, Sigma^{®}, Clontech^{®}, Cell Biolabs^{®}, SBI^{®}, Genecopoeia^{®} and many others. The methods are thus performed along with the guidelines supplied with the commercial kits.

In short, according to a non-limiting example taught by the γ-Retrovirus Guide on the website of Addgene, the following components are needed: (a) γ-Retroviral transfer plasmid encoding a transgene of interest: The transgene sequence is flanked by long terminal repeat (LTR) sequences, which facilitate integration of the transfer plasmid sequences into the host genome. Typically it is the sequences between and including the LTRs that is integrated into the host genome upon viral transduction; (b) Packaging genes (viral Gag-Pol): Gag is a structural precursor protein, and Pol is a polymerase; and (c) Envelope gene (may be pseudotyped to alter infectivity).

As a non-limiting example, the three components described above (envelope, packaging, and transfer) are supplied by three types of plasmids, which are cotransfected into a 293T packaging cell line. This system provides the greatest flexibility to pseudotype γ-retrovirus using different envelopes to modify tropism. Briefly, different envelope plasmids can direct the production of virus with various tropisms. A detailed non-limiting example of methods for preparation of recombinant retroviral stock and retroviral transduction of human CD4 T cells is found below in the Examples section.

In certain embodiments, the present invention provides a mammalian Treg expressing on its surface a homodimeric IL-10 as defined above for use in treating or preventing a disease, disorder or condition manifested in excessive or otherwise unwanted activity of the immune system, such as an autoimmune disease, allergy, asthma, and organ and bone marrow transplantation.

In yet another aspect, the present disclosure is directed to the mammalian Treg expressing on its surface a homodimeric IL-10 as defined above, for use in increasing immune suppression in a subject in need.

In certain embodiments, the mammalian Treg expressing on its surface a homodimeric IL-10 as defined above, are for use in treating or preventing a disease, disorder or condition, manifested in excessive or otherwise unwanted activity of the immune system.

In certain embodiments, the mammalian Treg is for treating a human subject and the mammalian Treg is a human Treg.

The specific diseases defined as autoimmune diseases are well known in the art; for example, as disclosed in The Encyclopedia of Autoimmune Diseases, Dana K. Cassell, Noel R. Rose, Infobase Publishing, 14 May 2014.

In certain embodiments, the autoimmune disease is selected from type 1 diabetes; rheumatoid arthritis; psoriasis; psoriatic arthritis; multiple sclerosis; systemic lupus erythematosus; inflammatory bowel disease, such as Crohn's disease and ulcerative colitis; Addison's disease; Graves' disease; Sjögren's syndrome; Hashimoto's thyroiditis; myasthenia gravis; vasculitis; pernicious anemia; celiac disease; and atherosclerosis.

In some embodiments, the subject is human and said mammalian Treg is human.

In some embodiments, Treg is an allogeneic Treg.

The stable Tr1 cells of the present invention may be used to increase immune suppression and treat diseases, disorders or conditions manifested in excessive or otherwise unwanted activity of the immune system without further genetic manipulation as evident from pre-clinical studies demonstrating that adoptive transfer of purified CD4⁺ CD25⁺ Tregs can inhibit or prevent disease in a range of models of autoimmune illness. These include, but are not restricted to systemic lupus erythematosus, inflammatory bowel disease, autoimmune encephalomyelitis, type 1 diabetes, autoimmune hepatitis and collagen-induced arthritis. Furthermore, adoptive transfer of these cells can protect against allograft rejection and graft versus host disease induced by allogeneic hematopoietic stem cell transplantation (13). In addition, a growing number of clinical trials evaluating the safety and efficacy of the adoptive transfer of ex-vivo-expanded, non-antigen-specific Tregs in the immunotherapy of a number of conditions and diseases, including graft-versus-host disease (GvHD), allograft rejection and type 1 diabetes (see (13) for review) show promise for this approach.

The beneficial clinical response observed in these studies may be improved in light of the cumulative evidence arguing that engagement of Tregs with antigen through their endogenous TCR enhances immune suppression (14-16).

The inventors of the present invention envision an approach in which the Tr1 cells are manipulated to express tissue-targeting proteins. For example, retinoic acid (RA) induces the expression of the gut-homing receptors integrin α4β7 and chemokine receptor CCR9 in T cells and can exert this function *in vivo* following pre-incubation *ex-vivo* (17, 18). RA is also a key regulator of TGF-β-mediated suppression by Tregs and promotes Treg differentiation (19). RA has also been shown to enhance the conversion of naive CD4 Teff cells into induced Tregs (20, 21) and to sustain Treg stability and function in the presence of IL-6 in an inflammatory environment (18). Preincubation with all-trans RA emerges as a feasible and simple procedure for equipping the reprogrammed Tr1 cells with gut homing capacity. The Tregs used in the methods for treating diseases as defined above may thus be contacted with retinoic acid prior to administration to the subject in order to equip the reprogrammed Tr1 cells with gut homing capacity and to sustain Treg stability and function in the presence of IL-6 in an inflammatory environment.

An attractive alternative solution capitalizes on the well-established ability to genetically redirect large numbers of T cells against cell surface antigens of choice using chimeric antigen receptors, or CARs (22).

In principle, CARs can also be used for reprogramming Tregs. Indeed, several laboratories have recently described the generation of functional mouse and human CAR-Tregs in different experimental settings ((23-29) and see (13, 16, 30, 31) for review). Redirecting Tregs through the transfer of exogenous TCR genes has also been reported (32-34).

A recent work in this field (28) has employed lentiviral transduction for generating HLA-A2-specific human CAR-Tregs as a means for preventing xenogeneic GvHD in immunodeficient mice caused by HLA-A2⁺ effector T cells. Indeed, *in-vivo* these CAR-Tregs were markedly superior to the same number CAR-Tregs of an irrelevant specificity in suppressing GvHD. The number of the HLA-A2 CAR-Tregs that were detectable in the blood of recipient mice peaked one week post-administration, remained stable for another week and then declined to near zero at the end of the third week.

Another example for the intended clinical use of CAR-Tregs has been reported recently, where retrovirally transduced human Tregs have been redirected at coagulation factor VIII (FVIII) in attempt to suppress the antibody response in replacement therapy for hemophilia A (29). Using a xenogeneic immunocompetent mouse model, strong suppression of the antibody response was evident 8 weeks post-immunization, although the introduced CAR-Tregs were already undetectable 2 weeks post-transfer.

Thus, the mammalian Tregs expressing on their surface a membrane-bound homodimeric IL-10 as defined herein and having a stable Tr1 phenotype are efficient agents for increasing immune suppression and treating diseases, disorders or conditions manifested in excessive or otherwise unwanted activity of the immune system; and agents that can be readily manipulated using techniques well-known in the art for increased efficacy. Furthermore, methods employing adoptive transfer of ex-vivo-expanded, non-antigen-specific as well as redirected antigen-specific Tregs are well known in the field of immunotherapy.

### Definitions

The term "Tr1 cells" is used interchangeably herein with the terms "iTregs" or "type 1 cells" and refers to CD4 T cells that are characterized by the expression of two cell surface markers, CD49b and LAG-3, low, or no expression of FoxP3, a non-proliferative (anergic) state, high production of IL-10 and TGF-β, but only minimally of IL-2 and none of IL-4 or IL-17, and the ability to suppress effector T cells (Teffs) in a cell-to-cell contact-independent manner.

The term "treating" as used herein refers to means of obtaining a desired physiological effect. The effect may be therapeutic in terms of partially or completely curing a disease and/or symptoms attributed to the disease. The term refers to inhibiting the disease, i.e. arresting its development; or ameliorating the disease, i.e. causing regression of the disease.

As used herein, the terms "subject" or "individual" or "animal" or "patient" or "mammal," refers to any subject, particularly a mammalian subject, for whom diagnosis, prognosis, or therapy is desired, for example, a human.

Pharmaceutical compositions for use in accordance with the present invention may be formulated in conventional manner using one or more physiologically acceptable carriers or excipients. The carrier(s) must be "acceptable" in the sense of being compatible with the other ingredients of the composition and not deleterious to the recipient thereof.

The following exemplification of carriers, modes of administration, dosage forms, etc., are listed as known possibilities from which the carriers, modes of administration, dosage forms, etc., may be selected for use with the present invention. Those of ordinary skill in the art will understand, however, that any given formulation and mode of administration selected should first be tested to determine that it achieves the desired results.

Methods of administration include, but are not limited to, parenteral, e.g., intravenous, intraperitoneal, intramuscular, subcutaneous, mucosal (e.g., oral, intranasal, buccal, vaginal, rectal, intraocular), intrathecal, topical and intradermal routes. Administration can be systemic or local. In certain embodiments, the pharmaceutical composition is adapted for oral administration.

The term "carrier" refers to a diluent, adjuvant, excipient, or vehicle with which the active agent is administered.

The term "variant" as used herein refers to polynucleotides or polypeptides modified at one or more base pairs, codons, introns, exons, or amino acid residues, respectively, yet still retain the biological activity of a polypeptide of the naturally occurring sequence

Unless otherwise indicated, all numbers expressing identity or similarity or any other parameter are to be understood as being modified in all instances by the term "about". Accordingly, unless indicated to the contrary, the numerical parameters set forth in this description and attached claims are approximations that may vary by up to plus or minus 10% depending upon the desired properties sought to be obtained by the present invention.

The invention will now be illustrated by the following non-limiting Examples.

### EXAMPLES

### Materials and Methods

### Separation of human CD4 T cells

Peripheral blood monocytes (PBMCs) have been prepared from whole blood samples or pheresis products using a standard Ficoll-Paque (Sigma) separation procedure. Twenty four hours post-separation (or after cell thawing) PBMCs were activated for 72 hours by plate-bound anti-CD3 Ab (OKT3) in the presence of soluble anti-CD28 and recombinant human IL-2. CD4 T cells were then separated using positive selection with magnetic beads (BD IMag^{™}) and then placed in complete medium for a 24 hour rest before experimental use.

### Preparation of recombinant retroviral stock

The memII,-10 gene was cloned into the commonly used MSGV1 retroviral vector via the BamHI-EcoRI restriction sites. The resulting plasmid, together with a plasmid carrying gag/pol and a plasmid carrying env were co-transfected to 3x10⁶ HEK293T cells placed in a 10 cm poly-D-lysine-coated plate in OptiMEM^{™} medium (a modification of Eagle's Minimum Essential Media, buffered with HEPES and sodium bicarbonate, and supplemented with hypoxanthine, thymidine, sodium pyruvate, L-glutamine, trace elements, and growth factor) with no antibiotics, using a transfection reagent such as either Lipofectamine (Thermo Fisher Scientific^{®}) or Fugene HD^{®} (Promega^{®}) according to the manufacturers' instructions. Next day cells were moved to complete medium with antibiotics and in the following day supernatant was collected and either frozen in aliquots or used directly for retroviral transduction.

### Retroviral transduction of human CD4 T cells

Transduction was performed in non-coated 6-well tissue culture plates. Wells were coated with a gene transduction enhancer (RetroNectin^{®}; Takara^{®}) overnight. RetroNectin^{®} is a 63 kD fragment of recombinant human fibronectin fragment (also referred to as rFN-CH-296) that enhances the efficiency of lentiviral- and retroviral-mediated gene transduction. RetroNectin^{®} was removed and wells were washed, blocked with 2.5% sterile bovine serum albumin (BSA) in phosphate buffered saline (PBS) and washed again. Viral supernatant was diluted in Dulbecco's Modified Eagle's medium (DMEM) containing a transfection reagent such as Polybrene (Merck^{®}) and moved to the RetroNectin^{®}-coated wells at 4 ml/well. Plates were centrifuged at 2000xg for 2 hours at 32°C, supernatant was aspirated and 4 ml of CD4 T cells at 5x105 cells/ml in 50/50 AIM-V/RPMI medium + 300 IU/ml recombinant IL-2 were added to each well. Plates were centrifuged for 15 minutes at 1000xg and incubated at 37°C overnight. CD4 T cells were then moved to new coated 6-well tissue culture plates and 1 ml of fresh 50/50 medium + 300 IU/ml rIL-2 was added to each well. In the following days medium was replaced and cells were split as needed.

### Example 1. Two IL-10 monomers linked together in tandem by a flexible linker and linked to a transmembrane-intracellular stretch via a short hinge region.

In the specific construct used here, two IL-10 monomers were linked together in tandem by a flexible linker of the sequence GSTSGSGKPGSGEGSTKG to create a homodimer, which was then linked to the transmembrane-intracellular stretch derived from the HLA-A2 heavy chain by a flexible hinge regions having a 21 amino acid spacer comprising the flexible linker

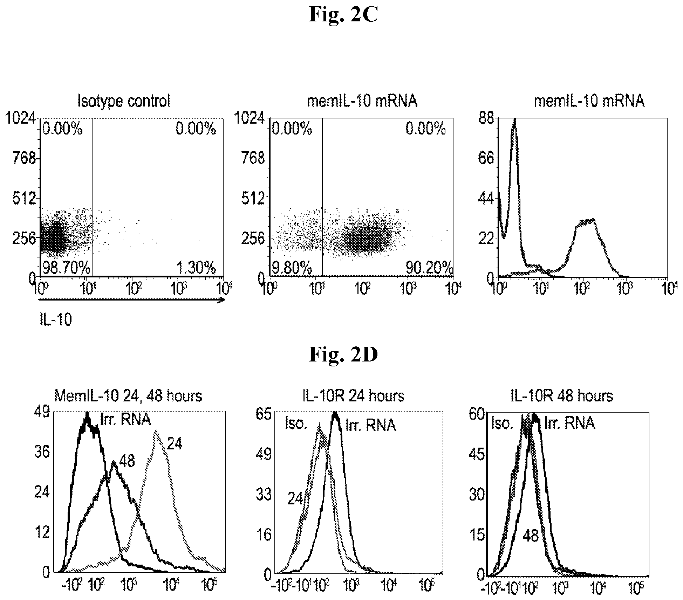

Gly₄Ser(Gly₃Ser)₂ and an additional 8 amino acid bridge of the sequence SSQPTIPI derived from the membrane-proximal part of the connecting peptide of HLA-A2 **(****Fig. 1****).** Surface expression of memIL-10 and IL-10R on human and mouse CD4 T cells was then confirmed **(****Fig. 2****).**

Elevation of the CD49b integrin could be observed in **(A)** and upregulation of IL-10 receptor (IL-10R) was similar to that induced by recombinant IL-10 (rIL-10, **(B)).** Mouse memIL-10 was clearly expressed 48 hours post-transfection **(D, left)** and, as expected, memIL-10 blocked the binding of the anti-mouse IL-10R mAb we used, suggesting binding in-cis (35).

### Example 2. Two IL-10 monomers linked together in tandem by a flexible linker and linked to a transmembrane-intracellular stretch via a long hinge region or the IL-10R β chain.

Our original memIL-10 constructs, both human and mouse, incorporated a hinge comprising a flexible linker of 21 amino acids (in addition to an 8 amino acid-long rigid spacer, now referred to herein as SmemIL-10 (S for short linker, see below).

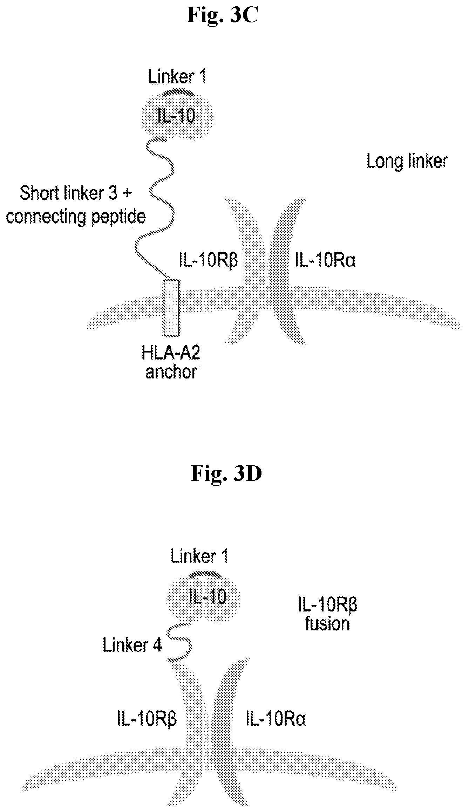

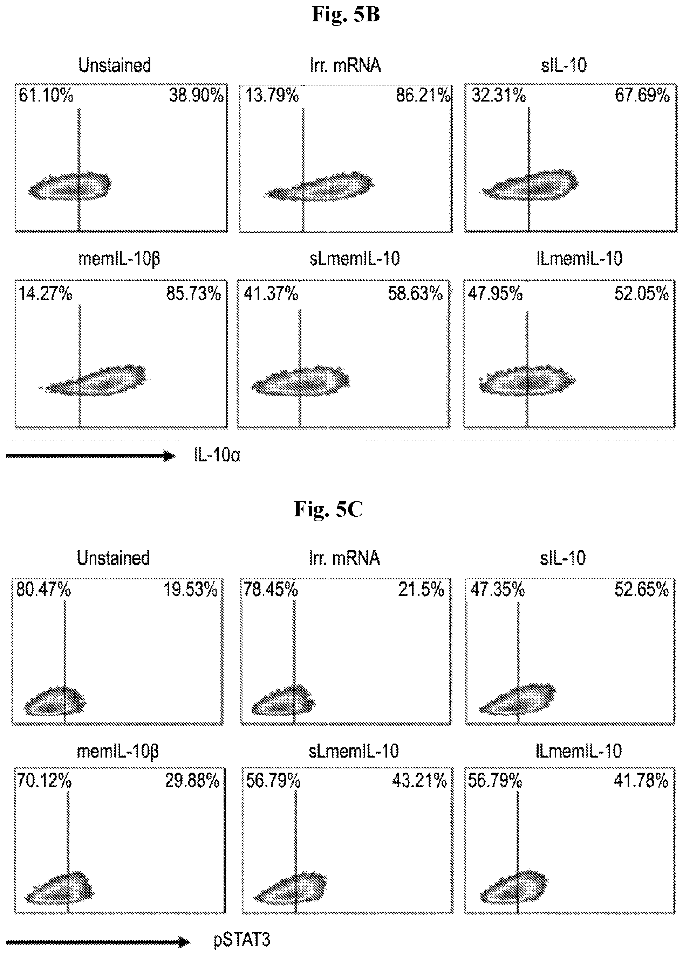

In attempt to optimize our memIL-10 we have engineered and cloned two new versions of this membrane cytokine: In one, cloned first, we provided memIL-10 with a longer linker peptide (of 30 amino acids, termed LmemIL-10 for long) to facilitate optimal engagement with IL-10R **(****Fig. 3****, lower left).** To create another derivative we fused our dimeric IL-10 to the N-terminus of the IL-10R β chain as a new scaffold designed to endow it with direct access to the IL-10 binding site located on the IL-10R α chain, designated memIL-10RB **(****Fig. 3****, lower right).** Indeed, **Fig. 4** confirms surface expression of the three products in human Jurkat cells. Of note, it is expected that the level of surface expression of the memIL-10RB fusion protein depends on the availability of IL-10Rα chain. To evaluate expression and function of the three different memIL-10 configurations mouse CD4 T cells were transfected with mRNA encoding the three constructs and assayed for surface expression (Fig. **5A****),** downregulation of surface IL-10R **(****Fig. 5B****)** and spontaneous phosphorylation of STAT3 **(****Fig. 5C****).** Indeed, in agreement with the results obtained in Jurkat cells, the constructs harboring the short and long linkers are expressed at much higher levels than memILL-10Rβ and exhibit superior function, as evident from the greater reduction in surface IL-10R and the stronger induction of pSTAT3. As the short linker construct (sLmemIL-10) was superior to the long linker one (lLmemIL-10) in its ability to induce pSTAT3 also in repeated experiments (not shown) it was selected for further experiments.

### Example 4. Expression and characterization of memIL-10 in retrovirally transduced mouse CD4 T cells.

To test expression and function of memIL-10 in retrovirally transduced T cells we first used splenic CD4 T cells purified with magnetic beads from C57BL/6 (B6) mice. As a negative control for memIL-10 transduced cells we used mock-transduced cells (Mock). Soluble IL-10 (sIL-10) was used in these experiments as a positive control. **Fig. 6** shows the results of a flow cytometry analysis of transduced cells vs. non-transduced ones which grew in the same culture and mock-transduced cells for the expression of the three Tr1-associated markers LAG-3, CD49b and PD-1 48 hours and 6 days post-transfection. Clear elevation of the 3 markers could indeed be observed already at day 2 which also persisted at day 6, pointing the expected phenotype. The ability of the transduced T cells to secrete IL-10 upon TCR-mediated activation confirmed the acquisition of Tr1-like functional properties **(****Fig. 7****).**

### Example 5. Assessing inhibitory effect of transduced cells on T effector cells.

To examine the ability of transduced cells to exert their inhibitory effect on neighboring Teff cells a coculture setting is designed which will allow us to selectively activate at will only one T cell population and not the other (obviously, anti-TCR/CD3 antibodies would activate all T cells in the coculture). To this end we will exploit two genes we have created, encoding the chimeric H-2K^{b}-CD3ζ (K^{b}-CD3ζ) and H-2K^{d}-CD3ζ (K^{d}-CD3ζ) MHC-I heavy chains. We have already shown that both genes selectively activate T cells following Ab-mediated cross-linking in magnitude that is comparable to TCR cross-linking. In the following series of functional experiments these tools are employed to mix mRNA-transfected Tr1 and Teff cells at different ratios for 3-4 days and use CFSE dilution and intracellular IFN-γ staining to assess the ability of activated Tr1 cells (vs. non-activated or RFP+ non-Tr1 cells) to suppress both proliferation and effector function of the activated Teffs.

### Example 6. Assessing in-vivo persistence of IL-10-transduced cells and suppressive function in mouse models for human diseases.

To evaluate in-vivo persistence of the IL-10-transduced NOD or B6 CD4 T cells in syngeneic wild-type mice and maintenance of their phenotype a protocol we recently established in our T1D experimental system (36) is used. Briefly, 10x106 cells are injected into the tail vain. Spleen and peripheral lymph nodes are harvested 1, 7 and 14 days post-injection and CD4+IL-10+LAG-3+CD49b+ T cells are identified by flow cytometry (compared to background level of staining in non-injected mice).

The actual suppressive function of memIL-10-tarsduced T cells under physiological conditions in-vivo is then tested, employing mouse models for human diseases such as T1D or IBD.

### Example 7. Expression and characterization of memIL-10 in retrovirally transduced human CD4 T cells.

For assessing the phenotypic and functional outcome of retroviral transduction of human CD4 T cells we isolated CD4 T cells from blood samples obtained from healthy donors through the Blood Services Center of Magen David Adom, Israel. The first of two independent *ex-vivo* experiments is presented in **Fig. 8****.** In this experiment cells have been kept in culture eighteen days post-transduction and phenotypic analyses for the markers LAG-3, CD49b, PD-1, 4-1BB, CD25 and IL-10Rα were performed by flow cytometry at days 1, 5 and 18 post-transduction. Our results confirm that all these cell surface markers that are associated with the expected Tr1 phenotype were significantly increased in memIL-10-expressing cells compared to memIL-10-negative cells that grew in the same culture dish for the entire period of the experiment.

The second experiment was performed on a different blood sample and flow cytometry performed for LAG-3, CD49b and PD-1 **(****Fig. 9****)** are in line with the results obtained in the first experiment. From these two experiments it can be concluded that long-term expression of memIL-10 in human CD4 T cells via retroviral transduction endows these cells with a TR-1-like phenotype.

### REFERENCES

1. Groux, H., A. O'Garra, M. Bigler, M. Rouleau, S. Antonenko, J. E. De Vries, and M. G. Roncarolo. 1997. A CD4+ T-cell subset inhibits antigen-specific T-cell responses and prevents colitis. Nature 389: 737-742.
2. Roncarolo, M. G., S. Gregori, R. Bacchetta, and M. Battaglia. 2014. Tr1 cells and the counter-regulation of immunity: Natural mechanisms and therapeutic applications. Curr. Top. Microbiol. Immunol. 380: 39-68.
3. Andolfi, G., G. Fousteri, M. Rossetti, C. F. Magnani, T. Jofra, G. Locafaro, A. Bondanza, S. Gregori, and M.-G. Roncarolo. 2012. Enforced IL-10 expression confers type 1 regulatory T cell (Tr1) phenotype and function to human CD4+ T cells. Mol. Ther. 20: 1778-1790.
4. Gagliani, N., C. F. Magnani, S. Huber, M. E. Gianolini, M. Pala, P. Licona-Limon, B. Guo, D. R. Herbert, A. Bulfone, F. Trentini, C. Di Serio, R. Bacchetta, M. Andreani, L. Brockmann, S. Gregori, R. A. Flavell, and M.-G. Roncarolo. 2013. Coexpression of CD49b and LAG-3 identifies human and mouse T regulatory type 1 cells. Nat. Med. 19: 739-746.
5. Zdanov, A., C. Schalk-Hihi, A. Gustchina, M. Tsang, J. Weatherbee, and A. Wlodawer. 1995. Crystal structure of interleukin-10 reveals the functional dimer with an unexpected topological similarity to interferon γ. Structure 3: 591-601.
6. Sabat, R., G. Grütz, K. Warszawska, S. Kirsch, E. Witte, K. Wolk, and J. Geginat. 2010. Biology of interleukin-10. IL-10 Fam. Cytokines 21: 331-344.
7. Chen, X., J. L. Zaro, and W.-C. Shen. 2013. Fusion protein linkers: property, design and functionality. Adv. Drug Deliv. Rev. 65: 1357-69.
8. Reddy Chichili, V. P., V. Kumar, and J. Sivaraman. 2013. Linkers in the structural biology of protein-protein interactions. Protein Sci. 22: 153-67.
9. Whitlow, M., B. A. Bell, S. L. Feng, D. Filpula, K. D. Hardman, S. L. Hubert, M. L. Rollence, J. F. Wood, M. E. Schott, and D. E. Milenic. 1993. An improved linker for single-chain Fv with reduced aggregation and enhanced proteolytic stability. Protein Eng. 6: 989-95.
10. Matuskova, M., and E. Durinikov. 2016. Retroviral Vectors in Gene Therapy. In Advances in Molecular Retrovirology InTech.
11. Abken, H. 2017. Driving CARs on the Highway to Solid Cancer: Some Considerations on the Adoptive Therapy with CAR T Cells. Hum. Gene Ther. 28: 1047-1060.
12. Zhou, X., S. Bailey-Bucktrout, L. T. Jeker, and J. A. Bluestone. 2009. Plasticity of CD4(+) FoxP3(+) T cells. Curr. Opin. Immunol. 21: 281-5.
13. Jethwa, H., A. A. Adami, and J. Maher. 2014. Use of gene-modified regulatory T-cells to control autoimmune and alloimmune pathology: Is now the right time? Clin. Immunol. 150: 51-63.
14. Levine, A. G., A. Arvey, W. Jin, and A. Y. Rudensky. 2014. Continuous requirement for the TCR in regulatory T cell function. Nat. Immunol. 15: 1070-1078.
15. Li, M. O., and A. Y. Rudensky. 2016. T cell receptor signalling in the control of regulatory T cell differentiation and function. Nat. Rev. Immunol. 16: 220-233.
16. Hoeppli, R. E., K. G. MacDonald, M. K. Levings, and L. Cook. 2016. How antigen specificity directs regulatory T-cell function: self, foreign and engineered specificity. HLA 88: 3-13.
17. Iwata, M., A. Hirakiyama, Y. Eshima, H. Kagechika, C. Kato, and S. Y. Song. 2004. Retinoic acid imprints gut-homing specificity on T cells. Immunity 21: 527-538.
18. Zhou, X., N. Kong, J. Wang, H. Fan, H. Zou, D. Horwitz, D. Brand, Z. Liu, and S. G. Zheng. 2010. Cutting edge: all-trans retinoic acid sustains the stability and function of natural regulatory T cells in an inflammatory milieu. J Immunol 185: 2675-2679.
19. Mucida, D., Y. Park, G. Kim, O. Turovskaya, I. Scott, M. Kronenberg, and H. Cheroutre. 2007. Reciprocal TH17 and regulatory T cell differentiation mediated by retinoic acid. Science (80-. ). 317: 256-260.
20. Wang, J., T. W. Huizinga, and R. E. Toes. 2009. De novo generation and enhanced suppression of human CD4+CD25+ regulatory T cells by retinoic acid. J Immunol 183: 4119-4126.
21. Nolting, J., C. Daniel, S. Reuter, C. Stuelten, P. Li, H. Sucov, B. G. Kim, J. J. Letterio, K. Kretschmer, H. J. Kim, and H. von Boehmer. 2009. Retinoic acid can enhance conversion of naive into regulatory T cells independently of secreted cytokines. J Exp Med 206: 2131-2139.
22. Gross, G., and Z. Eshhar. 2016. Therapeutic Potential of T Cell Chimeric Antigen Receptors (CARs) in Cancer Treatment: Counteracting Off-Tumor Toxicities for Safe CAR T Cell Therapy. Annu. Rev. Pharmacol. Toxicol. 56: 59-83.
23. Elinav, E., T. Waks, and Z. Eshhar. 2008. Redirection of regulatory T cells with predetermined specificity for the treatment of experimental colitis in mice. Gastroenterology 134: 2014-2024.
24. Elinav, E., N. Adam, T. Waks, and Z. Eshhar. 2009. Amelioration of colitis by genetically engineered murine regulatory T cells redirected by antigen-specific chimeric receptor. Gastroenterology 136: 1721-1731.
25. Hombach, A. A., D. Kofler, G. Rappl, and H. Abken. 2009. Redirecting human CD4+CD25+ regulatory T cells from the peripheral blood with pre-defined target specificity. Gene Ther 16: 1088-1096.
26. Lee, J. C., E. Hayman, H. J. Pegram, E. Santos, G. Heller, M. Sadelain, and R. Brentjens. 2011. In vivo inhibition of human CD19-targeted effector T cells by natural T regulatory cells in a xenotransplant murine model of B cell malignancy. Cancer Res. 71: 2871-2881.
27. Blat, D., E. Zigmond, Z. Alteber, T. Waks, and Z. Eshhar. 2014. Suppression of murine colitis and its associated cancer by carcinoembryonic antigen-specific regulatory T cells. Mol. Ther. 22: 1018-1028.
28. MacDonald, K. G., R. E. Hoeppli, Q. Huang, J. Gillies, D. S. Luciani, P. C. Orban, R. Broady, and M. K. Levings. 2016. Alloantigen-specific regulatory T cells generated with a chimeric antigen receptor. J. Clin. Invest. 126: 1413-24.
29. Yoon, J., A. Schmidt, A.-H. Zhang, C. Königs, Y. C. Kim, and D. W. Scott. 2017. FVIII-specific human chimeric antigen receptor T-regulatory cells suppress T- and B-cell responses to FVIII. Blood 129: 238-245.
30. Maldini, C. R., G. I. Ellis, and J. L. Riley. 2018. CAR T cells for infection, autoimmunity and allotransplantation. Nat. Rev. Immunol. 18: 605-616.
31. Zhang, Q., W. Lu, C.-L. Liang, Y. Chen, H. Liu, F. Qiu, and Z. Dai. 2018. Chimeric Antigen Receptor (CAR) Treg: A Promising Approach to Inducing Immunological Tolerance. Front. Immunol. 9: 2359.
32. Wright, G. P., C. A. Notley, S. A. Xue, G. M. Bendle, A. Holler, T. N. Schumacher, M. R. Ehrenstein, and H. J. Stauss. 2009. Adoptive therapy with redirected primary regulatory T cells results in antigen-specific suppression of arthritis. Proc Natl Acad Sci U S A 106: 19078-19083.
33. Brusko, T. M., R. C. Koya, S. Zhu, M. R. Lee, A. L. Putnam, S. A. McClymont, M. I. Nishimura, S. Han, L. J. Chang, M. A. Atkinson, A. Ribas, and J. A. Bluestone. 2010. Human antigen-specific regulatory T cells generated by T cell receptor gene transfer. PLoS One 5.
34. Wan, Q., L. Kozhaya, K. Imberg, F. Mercer, S. Zhong, M. Krogsgaard, and D. Unutmaz. 2013. Probing the effector and suppressive functions of human T cell subsets using antigen-specific engineered T cell receptors. PLoS One 8: e56302.
35. Weinstein-Marom, H., A. Pato, N. Levin, K. Susid, O. Itzhaki, M. J. Besser, T. Peretz, A. Margalit, M. Lotem, and G. Gross. 2016. Membrane-attached Cytokines Expressed by mRNA Electroporation Act as Potent T-Cell Adjuvants. J. Immunother. 39: 60-70.
36. Lewis, M. D., E. de Leenheer, S. Fishman, L. K. Siew, G. Gross, and F. S. Wong. 2015. A reproducible method for the expansion of mouse CD8+ T lymphocytes. J. Immunol. Methods 417: 134-138.

## Claims

1. A nucleic acid molecule comprising a nucleotide sequence encoding a membrane-bound homodimeric IL-10 (mem-IL10) comprising a homodimeric IL-10 linked to a transmembrane-intracellular stretch.

2. The nucleic acid molecule of claim 1, wherein:
(i) said homodimeric IL-10 is linked to said transmembrane-intracellular stretch through a flexible hinge, and/or
(ii) said homodimeric IL-10 comprises a first and a second IL-10 monomer connected in a single-chain configuration such that the C-terminus of the first IL-10 monomer is linked to the N-terminus of the second IL-10 monomer via a first flexible linker, wherein, optionally, said first flexible linker has the amino acid sequence GSTSGSGKPGSGEGSTKG (SEQ ID NO: 1).

3. The nucleic acid molecule of claim 2, wherein said flexible hinge comprises a polypeptide selected from a hinge region of CD8α, a hinge region of a heavy chain of IgG, a hinge region of a heavy chain of IgD; an extracellular stretch of an IL-10R β chain; and a second flexible linker comprising an amino acid sequence of up to 28 amino acids comprising at least one Gly4Ser(Gly3 Ser)₂ sequence, wherein, optionally:
(i) said second flexible hinge comprises a 21 amino acid sequence comprising the amino acid sequence Gly₄Ser(Gly₃Ser)₂ (referred to herein as "short linker"; SEQ ID NO: 13), or
(ii) said second flexible linker comprises a 28 amino acid sequence comprising the amino acid sequence Gly₄Ser(Gly₃Ser)₂Ser₂(Gly₃Ser)₃ (referred to herein as "long linker"; SEQ ID NO: 15).

4. The nucleic acid molecule of claim 3, wherein said polypeptide further comprises an amino acid bridge of the sequence SSQPTIPI (SEQ ID NO: 17).

5. The nucleic acid molecule of any one of claims 1 to 4, wherein said transmembrane-intracellular stretch is derived from a heavy chain of a human MHC class I molecule selected from an HLA-A, HLA-B or HLA-C molecule, preferably HLA-A2; human CD28; or human IL-10R β chain.

6. The nucleic acid molecule of any one of claims 1 to 5, wherein the amino acid sequence of the complete mem-IL10 comprises or essentially consists of the homodimeric IL-10 linked via:
(i) the short second flexible linker and the connecting peptide to the transmembrane-intracellular stretch of HLA-A2 as set forth in SEQ ID NO: 25, or
(ii) the long second flexible linker and the connecting peptide to the transmembrane-intracellular stretch of HLA-A2 as set forth in SEQ ID NO: 27.

7. The nucleic acid molecule of any one of claims 1 to 5, wherein the homodimeric IL-10 is linked to the N-terminus of an essentially complete human IL-10R β chain via the short linker as set forth in SEQ ID NO: 29.

8. A homodimeric IL-10 linked to a transmembrane-intracellular stretch, as encoded by the nucleic acid of any one of claims 1-7.

9. A composition comprising the nucleic acid molecule of any one of claims 1 to 7.

10. A viral vector comprising the nucleic acid molecule of any one of claims 1 to 7, which, optionally, is a modified virus derived from a virus selected from a retrovirus, lentivirus, gammavirus, adenovirus, adeno-associated virus, pox virus, alphavirus, and herpes virus.

11. A composition comprising the viral vector of claim 10.

12. A mammalian regulatory T cell (Treg) which comprises the nucleic acid molecule of any one of claims 1 to 7 or the viral vector of claim 9, and/or expresses on its surface a homodimeric IL-10 that is linked to a transmembrane-intracellular stretch, wherein optionally the Treg
(i) is a human Treg,
(ii) has a stable Tr1 phenotype exhibiting the cell-surface markers CD49b and LAG-3, and, optionally, further exhibits PD-1, 4-1BB, CD25 and IL-10Rα; and/or
(iii) is manipulated to express a tissue-targeting protein or a chimeric antigen receptor.

13. An ex vivo method of preparing allogeneic or autologous Tregs with a stable Tr1 phenotype, the method comprising contacting CD4 T cells with the nucleic acid molecule of any one of claims 1 to 7, a viral vector comprising it or the homodimeric IL-10 of claim 8, thereby endowing said CD4 T cells with a stable Tr1 phenotype, and thus preparing Tregs with a stable Tr1 phenotype.

14. The mammalian Treg of claim 12, for use in treating or preventing a disease, disorder or condition, manifested in excessive or unwanted activity of the immune system, optionally wherein said disease, disorder or condition is selected from an autoimmune disease, allergy, asthma, and organ and bone marrow transplantation, further optionally wherein the autoimmune disease is selected from type 1 diabetes; rheumatoid arthritis; psoriasis; psoriatic arthritis; multiple sclerosis; systemic lupus erythematosus; inflammatory bowel disease, such as Crohn's disease and ulcerative colitis; Addison's disease; Graves' disease; Sjögren's syndrome; Hashimoto's thyroiditis; myasthenia gravis; vasculitis; pernicious anemia; celiac disease; and atherosclerosis.

15. The mammalian Treg for the use of claim 14, wherein said mammalian Treg is a human Treg and is used for treating or preventing said disease, disorder or condition in a human subject, optionally wherein said human Treg is an allogeneic Treg.

## Patentansprüche

1. Nukleinsäuremolekül, umfassend eine Nukleotidsequenz, die ein membrangebundenes homodimeres IL-10 (mem-IL10) kodiert, umfassend ein homodimeres IL-10, das an einen Transmembran-Intrazellulär-Abschnitt gebunden ist.

2. Nukleinsäuremolekül nach Anspruch 1, wobei
(i) das homodimere IL-10 über ein flexibles Gelenk an den Transmembran-Intrazellulär-Abschnitt gebunden ist, und/oder
(ii) das homodimere IL-10 ein erstes und ein zweites IL-10-Monomer umfasst, die in einer einkettigen Konfiguration so verbunden sind, dass der C-Terminus des ersten IL-10-Monomers über einen ersten flexiblen Linker an den N-Terminus des zweiten IL-10-Monomers gebunden ist, wobei, optional, der erste flexible Linker die Aminosäuresequenz GSTSGSGKPGSGEGSTKG (SEQ ID NO: 1) aufweist.

3. Nukleinsäuremolekül nach Anspruch 2, wobei das flexible Gelenk ein Polypeptid umfasst, das ausgewählt ist aus einem Gelenkbereich von CD8α, einem Gelenkbereich einer schweren Kette von IgG, einem Gelenkbereich einer schweren Kette von IgD; einem extrazellulären Abschnitt einer IL-10R-β-Kette; und einem zweiten flexiblen Linker, der eine Aminosäuresequenz von bis zu 28 Aminosäuren umfasst, die mindestens eine Gly4Ser(Gly3Ser)₂-Sequenz umfasst, wobei, optional:
(i) das zweite flexible Gelenk eine Sequenz mit 21 Aminosäuren umfasst, die die Aminosäuresequenz Gly₄Ser(Gly₃Ser)₂ (hierin als "kurzer Linker" bezeichnet; SEQ ID NO: 13) umfasst, oder
(ii) der zweite flexible Linker eine Sequenz mit 28 Aminosäuren umfasst, die die Aminosäuresequenz Gly₄Ser(Gly₃Ser)₂Ser₂(Gly₃Ser)₃ (hierin als "langer Linker" bezeichnet; SEQ ID NO: 15) umfasst.

4. Nukleinsäuremolekül nach Anspruch 3, wobei das Polypeptid ferner eine Aminosäurebrücke der Sequenz SSQPTIPI (SEQ ID NO: 17) umfasst.

5. Nukleinsäuremolekül nach einem der Ansprüche 1 bis 4, wobei der Transmembran-Intrazellulär-Abschnitt von einer schweren Kette eines humanen MHC-Klasse-I-Moleküls abgeleitet ist, das aus einem HLA-A-, HLA-B- oder HLA-C-Molekül, bevorzugt HLA-A2; humanes CD28; oder humane IL-10R-β-Kette, ausgewählt ist.

6. Nukleinsäuremolekül nach einem der Ansprüche 1 bis 5, wobei die Aminosäuresequenz des vollständigen mem-IL10 das homodimere IL-10 umfasst oder im Wesentlichen daraus besteht, verbunden über:
(i) den kurzen zweiten flexiblen Linker und das verbindende Peptid mit dem Transmembran-Intrazellulär-Abschnitt von HLA-A2, wie in SEQ ID NO: 25 angegeben, oder
(ii) den langen zweiten flexiblen Linker und das verbindende Peptid mit dem Transmembran-Intrazellulär-Abschnitt von HLA-A2, wie in SEQ ID NO: 27 angegeben.

7. Nukleinsäuremolekül nach einem der Ansprüche 1 bis 5, wobei das homodimere IL-10 über den kurzen Linker an den N-Terminus einer im Wesentlichen vollständigen humanen IL-10R-β-Kette gebunden ist, wie in SEQ ID NO: 29 angegeben.

8. Homodimeres IL-10, das an einen Transmembran-Intrazellulär-Abschnitt gebunden ist, wie durch die Nukleinsäure nach einem der Ansprüche 1 bis 7 kodiert.

9. Zusammensetzung, umfassend das Nukleinsäuremolekül nach einem der Ansprüche 1 bis 7.

10. Viraler Vektor, der das Nukleinsäuremolekül nach einem der Ansprüche 1 bis 7 umfasst, der, optional, ein modifiziertes Virus ist, das von einem Virus abgeleitet ist, das aus einem Retrovirus, Lentivirus, Gammavirus, Adenovirus, Adeno-assoziierten Virus, Pockenvirus, Alphavirus und Herpesvirus ausgewählt ist.

11. Zusammensetzung, umfassend den viralen Vektor nach Anspruch 10.

12. Regulatorische Säugetier-T-Zelle (Treg), die das Nukleinsäuremolekül nach einem der Ansprüche 1 bis 7 oder den viralen Vektor nach Anspruch 9 umfasst und/oder auf ihrer Oberfläche ein homodimeres IL-10 exprimiert, das an einen Transmembran-Intrazellulär-Abschnitt gebunden ist, wobei optional die Treg
(i) eine humane Treg ist,
(ii) einen stabilen Tr1-Phänotyp aufweist, der die Zelloberflächenmarker CD49b und LAG-3 zeigt, und, optional, ferner PD-1, 4-1BB, CD25 und IL-10Rα zeigt; und/oder
(iii) so manipuliert ist, dass sie ein gewebespezifisches Protein oder einen chimären Antigenrezeptor exprimiert.

13. Ex-vivo-Verfahren zur Herstellung allogener oder autologer Tregs mit einem stabilen Tr1-Phänotyp, wobei das Verfahren ein Inkontaktbringen von CD4-T-Zellen mit dem Nukleinsäuremolekül nach einem der Ansprüche 1 bis 7, einem viralen Vektor, der dieses umfasst, oder dem homodimeren IL-10 nach Anspruch 8 umfasst, wodurch die CD4-T-Zellen mit einem stabilen Tr1-Phänotyp ausgestattet werden und somit Tregs mit einem stabilen Tr1-Phänotyp hergestellt werden.

14. Säugetier-Treg nach Anspruch 12, zur Verwendung bei der Behandlung oder Vorbeugung einer Krankheit, Störung oder eines Zustands, die bzw. der sich in einer übermäßigen oder unerwünschten Aktivität des Immunsystems manifestiert, optional wobei die Krankheit, Störung oder der Zustand aus einer Autoimmunerkrankung, einer Allergie, Asthma und einer Organ- und Knochenmarktransplantation ausgewählt ist, ferner optional wobei die Autoimmunerkrankung aus Typ-1-Diabetes; rheumatoider Arthritis; Psoriasis; Psoriasis-Arthritis; Multipler Sklerose; systemischem Lupus erythematodes; entzündlicher Darmerkrankung wie Morbus Crohn und Colitis ulcerosa; Morbus Addison; Morbus Basedow; Sjögren-Syndrom; Hashimoto-Thyreoiditis; Myasthenia gravis; Vaskulitis; perniziöser Anämie; Zöliakie und Atherosklerose ausgewählt ist.

15. Säugetier-Treg für die Verwendung nach Anspruch 14, wobei die Säugetier-Treg eine humane Treg ist und zur Behandlung oder Vorbeugung der Krankheit, Störung oder des Zustands bei einem humanen Patienten verwendet wird, optional wobei die humane Treg eine allogene Treg ist.

## Revendications

1. Molécule d'acide nucléique comportant une séquence nucléotidique codant pour une IL-10 homodimérique liée à une membrane (mem-IL10) comportant une IL-10 homodimérique liée à un étirement transmembranaire-intracellulaire.

2. Molécule d'acide nucléique selon la revendication 1, dans laquelle :
(i) ladite IL-10 homodimérique est liée audit étirement transmembranaire-intracellulaire par le biais d'une charnière flexible, et/ou
(ii) ladite IL-10 homodimérique comporte un premier et un deuxième monomère IL-10 reliés dans une configuration monocaténaire de sorte que l'extrémité C-terminale du premier monomère d'IL-10 soit liée à l'extrémité N-terminale du deuxième monomère d'IL-10 via un premier lieur flexible, dans laquelle, facultativement, ledit premier lieur flexible présente la séquence d'acides aminés GSTSGSGKPGSGEGSTKG (SEQ ID NO : 1).

3. Molécule d'acide nucléique selon la revendication 2, dans laquelle ladite charnière flexible comporte un polypeptide choisi parmi une région charnière de CD8α, une région charnière d'une chaîne lourde d'IgG, une région charnière d'une chaîne lourde d'IgD ; un étirement extracellulaire d'une chaîne β d'IL-10R ; et un deuxième lieur flexible comportant une séquence d'acides aminés allant jusqu'à 28 acides aminés comportant au moins une séquence Gly4Ser(Gly3Ser)₂, dans laquelle, facultativement :
(i) ladite deuxième charnière flexible comporte une séquence de 21 acides aminés comportant la séquence d'acides aminés Gly₄Ser(Gly₃Ser)₂ (ci-après dénommée « lieur court » ; SEQ ID NO : 13), ou
(ii) ledit deuxième lieur flexible comporte une séquence de 28 acides aminés comportant la séquence d'acides aminés Gly₄Ser(Gly₃Ser)₂Ser₂(Gly₃Ser)₃ (ci-après dénommée « lieur long » ; SEQ ID NO : 15).

4. Molécule d'acide nucléique selon la revendication 3, dans laquelle ledit polypeptide comporte en outre un pont d'acides aminés de la séquence SSQPTIPI (SEQ ID NO : 17).

5. Molécule d'acide nucléique selon l'une quelconque des revendications 1 à 4, dans laquelle ledit étirement transmembranaire-intracellulaire est dérivé d'une chaîne lourde d'une molécule MHC de classe I humaine sélectionnée parmi une molécule HLA-A, HLA-B ou HLA-C, de préférence HLA-A2 ; CD28 humain ; ou une chaîne β IL-10R humaine.

6. Molécule d'acide nucléique selon l'une quelconque des revendications 1 à 5, dans laquelle la séquence d'acides aminés de la mem-IL10 complète comporte ou consiste essentiellement en l'IL-10 homodimérique liée via :
(i) le deuxième lieur flexible court et le peptide de connexion à l'étirement transmembranaire-intracellulaire de HLA-A2 tel que représenté dans SEQ ID NO : 25, ou
(ii) le deuxième lieur flexible long et le peptide de connexion à l'étirement transmembranaire-intracellulaire de HLA-A2 tel que représenté dans SEQ ID NO : 27.

7. Molécule d'acide nucléique selon l'une quelconque des revendications 1 à 5, dans laquelle l'IL-10 homodimérique est liée à l'extrémité N-terminale d'une chaîne β d'IL-10R humaine essentiellement complète via le lieur court tel que représenté dans SEQ ID NO : 29.

8. IL-10 homodimérique liée à un étirement transmembranaire-intracellulaire, telle que codée par l'acide nucléique selon l'une quelconque des revendications 1 à 7.

9. Composition comportant la molécule d'acide nucléique selon l'une quelconque des revendications 1 à 7.

10. Vecteur viral comportant la molécule d'acide nucléique selon l'une quelconque des revendications 1 à 7, qui est, facultativement, un virus modifié dérivé d'un virus sélectionné parmi un rétrovirus, un lentivirus, un gammavirus, un adénovirus, un virus adénoassocié, le virus de la variole, un alphavirus et le virus de l'herpès.

11. Composition comportant le vecteur viral selon la revendication 10.

12. Lymphocyte T régulateur (Treg) de mammifère qui comporte la molécule d'acide nucléique selon l'une quelconque des revendications 1 à 7 ou le vecteur viral selon la revendication 9, et/ou exprime sur sa surface une IL-10 homodimérique qui est liée à un étirement transmembranaire-intracellulaire, dans lequel facultativement le Treg
(i) est un Treg humain,
(ii) présente un phénotype Tr1 stable présentant les marqueurs antigéniques CD49b et LAG-3, et, facultativement, présente en outre PD-1, 4-1BB, CD25 et IL-10Rα ; et/ou
(iii) est manipulé pour exprimer une protéine de ciblage tissulaire ou un récepteur antigénique chimérique.

13. Procédé ex vivo de préparation de Tregs allogéniques ou autologues avec un phénotype Tr1 stable, le procédé comportant la mise en contact de lymphocytes T CD4 avec la molécule d'acide nucléique selon l'une quelconque des revendications 1 à 7, un vecteur viral comportant celle-ci ou l'IL-10 homodimérique selon la revendication 8, pour ainsi doter lesdits lymphocytes T CD4 d'un phénotype Tr1 stable et ainsi préparer des Tregs avec un phénotype Tr1 stable.

14. Treg de mammifère selon la revendication 12, pour une utilisation dans le traitement ou la prévention d'une maladie, d'un trouble ou d'une affection, se manifestant par une activité excessive ou indésirable du système immunitaire, facultativement dans lequel ladite maladie, ledit trouble ou ladite affection est choisi(e) parmi une maladie auto-immune, une allergie, un asthme, et une greffe d'organe et de moelle osseuse, en outre facultativement dans lequel la maladie auto-immune est choisie parmi le diabète de type 1 ; la polyarthrite rhumatoïde ; le psoriasis ; l'arthrite psoriasique ; la sclérose en plaques ; le lupus érythémateux disséminé ; une maladie inflammatoire de l'intestin, telle que la maladie de Crohn et la rectocolite hémorragique ; la maladie d'Addison ; la maladie de Graves ; le syndrome de Sjögren ; la thyroïdite d'Hashimoto ; la myasthénie grave ; la vascularite ; l'anémie pernicieuse ; la maladie cœliaque ; et l'athérosclérose.

15. Treg de mammifère pour l'utilisation selon la revendication 14, dans lequel ledit Treg de mammifère est un Treg humain et est utilisé pour traiter ou prévenir ladite maladie, ledit trouble ou ladite affection chez un sujet humain, facultativement dans lequel ledit Treg humain est un Treg allogénique.
